# EUROPEAN PATENT APPLICATION

(11) **EP 4 628 588 A1**
(43) Date of publication of application: **08.10.2025**
(21) Application number: 23913025.5
(22) Date of filing: 29.12.2023
(51) Int. Cl.: C12N 15/70, C12N 9/18, A23K 10/16

(54) **FUMONISIN DEGRADATION ENZYME WITH IMPROVED ACTIVITY, AND USE THEREOF**

(30) Priority: 29.12.2022 KR 20220189271
(71) Applicant: CJ CheilJedang Corporation, Seoul 04560 (KR)
(72) Inventor: KANG, Young Seo, Seoul 04560 (KR); LEE, Seung-hee, Seoul 04560 (KR); KIM, Nam Yoon, Seoul 04560 (KR); YANG, Tae Joo, Seoul 04560 (KR); OH, Kyoungmi, Seoul 04560 (KR); SHIM, Jihyun, Seoul 04560 (KR); HONG, So Yeon, Seoul 04560 (KR)
(74) Representative: Meissner Bolte Partnerschaft mbB
(86) International application number: PCT/KR2023/021988
(87) International publication number: WO 2024/144351

(57) **Abstract**

Provided are a fumonisin degradation enzyme with improved activity and use thereof.

## Description

### [Technical Field]

The present disclosure relates to a fumonisin degradation enzyme with improved activity and use thereof.

### [Background Art]

Fumonisin is a mycotoxin predominantly produced by *Fusarium verticillioides* and *Fusarium proliferatum* which are plant pathogenic fungi. These fungi often contaminate corn or corn-based products, and cause various diseases when ingested by humans or animals through food or feed. Generally, fumonisin is known to have hepatotoxicity and nephrotoxicity in animals, and is known to be related to esophageal cancer and neural tube defects in humans. For this reason, fumonisin B1, which is the most frequently found among fumonisins, is classified by the International Agency for Research on Cancer (IARC) as Group 2B, possibly carcinogenic to humans.

Accordingly, there is a need for an effective composition or method for detoxifying fumonisin present in corn or corn-based products.

### [Disclosure]

### [Technical Problem]

The present disclosure relates to a fumonisin degradation enzyme with improved activity and use thereof.

### [Technical Solution]

An object of the present disclosure is to provide a polypeptide having fumonisin degradation activity.

Another object of the present disclosure is to provide a polynucleotide encoding the polypeptide.

Still another object of the present disclosure is to provide a vector comprising the polynucleotide.

Still another object of the present disclosure is to provide a composition for degrading fumonisin, the composition comprising any one or more of a polypeptide having fumonisin degradation activity; a polynucleotide encoding the polypeptide; a vector comprising the polynucleotide; and a host cell expressing the polypeptide.

Still another object of the present disclosure is to provide a composition for detoxifying fumonisin that is present in food, feed, or both thereof, the composition comprising any one or more of a polypeptide having fumonisin degradation activity; a polynucleotide encoding the polypeptide; a vector comprising the polynucleotide; and a host cell expressing the polypeptide.

Still another object of the present disclosure is to provide a feed additive composition comprising any one or more of a polypeptide having fumonisin degradation activity; a polynucleotide encoding the polypeptide; a vector comprising the polynucleotide; and a host cell expressing the polypeptide.

Still another object of the present disclosure is to provide a method of degrading fumonisin, the method comprising the step of bringing fumonisin into contact with any one or more of a polypeptide having fumonisin degradation activity; and a host cell expressing the polypeptide.

Still another object of the present disclosure is to provide a method of preparing a polypeptide having fumonisin degradation activity, the method comprising the step of culturing a host cell comprising any one or more of a polypeptide having fumonisin degradation activity, a polynucleotide encoding the polypeptide, and a vector comprising the polynucleotide.

Still another object of the present disclosure is to provide use of a polypeptide having SEQ ID NO: 3 or SEQ ID NO: 5, or 75% or more sequence identity thereto, as a fumonisin degradation enzyme.

### [Advantageous Effects]

A mycotoxin, fumonisin may be effectively degraded by using a polypeptide having fumonisin degradation activity of the present disclosure.

### [Brief Description of the Drawing]

FIG. 1 shows the results of sequence alignment of carboxyesterase "EFB1-9" (SEQ ID NO: 1) derived from a microorganism of *Bradyrhizobium* sp., and two types of variants comprising multiple mutations, "CB1" (SEQ ID NO: 3) and "CB4" (SEQ ID NO: 5); and
FIG. 2 shows the results of examining the fumonisin degradation enzyme activity of EFB1-9, CB1, and CB4 using thin layer chromatography (TLC).

### [Detailed Description of Preferred Embodiments]

The present disclosure will be described in detail as follows. Meanwhile, each description and embodiment disclosed in this disclosure may also be applied to other descriptions and embodiments. That is, all combinations of various elements disclosed in this disclosure fall within the scope of the present disclosure. Further, the scope of the present disclosure is not limited by the specific description described below.

Further, those skilled in the art will recognize, or be able to ascertain using no more than routine experimentation, many equivalents to the specific embodiments of the disclosure described herein. Further, these equivalents should be interpreted to fall within the present disclosure.

Further, a number of papers and patent documents are referenced and cited throughout this specification. The disclosures of the cited papers and patent documents are incorporated herein by reference in their entirety to further clarify the level and scope of the subject matter to which the present disclosure pertains.

One aspect of the present disclosure provides a polypeptide of SEQ ID NO: 3 having fumonisin degradation activity.

One aspect of the present disclosure provides a polypeptide of SEQ ID NO: 5 having fumonisin degradation activity.

The polypeptide may also be referred to as a fumonisin degradation enzyme.

Meanwhile, the degradation of fumonisin may be used in the same meaning as detoxification of fumonisin, inactivation of fumonisin, and removal of fumonisin contamination (decontaminating).

Fumonisin of the present disclosure comprises fumonisin B1, fumonisin B2, fumonisin B3, fumonisin B4, fumonisin A1 and fumonisin A2; and derivatives thereof. For example, fumonisin of the present disclosure may be selected from fumonisin B1, fumonisin B2, fumonisin B3, fumonisin B4, fumonisin A1, and fumonisin A2. For example, fumonisin of the present disclosure may be fumonisin B1.

Fumonisin B1 (FB1) and a hydrolysate thereof, HFB1, may have a structure shown in Chemical Formula 1 below.

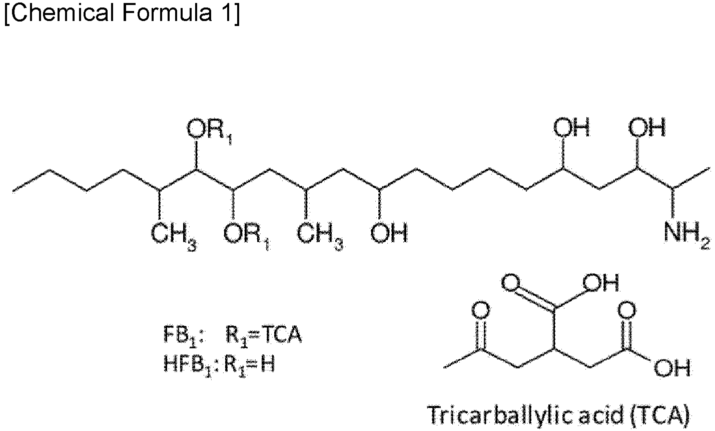

For example, the fumonisin degradation activity may be determined by detecting the hydrolysate of fumonisin.

Meanwhile, although the novel polypeptide having fumonisin degradation activity provided in the present disclosure is defined as a polypeptide of SEQ ID NO: 3 or SEQ ID NO: 5, the addition of a meaningless sequence upstream or downstream of the amino acid sequence of SEQ ID NO: 3 or SEQ ID NO: 5, a naturally occurring mutation, or a silent mutation thereof or conservative substitution is not excluded, and it will be apparent to those skilled in the art that as long as a protein has activity identical or corresponding to the activity of the protein consisting of the amino acid sequence of SEQ ID NO: 3 or SEQ ID NO: 5, it may belong to the polypeptide having fumonisin degradation activity provided in the present disclosure.

In other words, although described as 'a protein or polypeptide of an amino acid sequence represented by a specific sequence number', 'a protein or polypeptide having an amino acid sequence represented by a specific sequence number', or 'a protein or polypeptide comprising an amino acid sequence represented by a specific sequence number' in the present disclosure, it is obvious that any protein having an amino acid sequence with deletion, modification, substitution, or addition in part of the sequence may also be used in the present disclosure, as long as the protein may have an activity identical or corresponding to that of the polypeptide consisting of the amino acid sequence of the corresponding sequence number.

Further, a variant polypeptide, which differs from the recited sequence in one or more amino acids by conservative substitution and/or modification, as compared to the amino acid sequence of SEQ ID NO: 3 or SEQ ID NO: 5, but retains the functions or properties of the protein, also falls within the scope of the polypeptide provided in the present disclosure. Such modifications may comprise, for example, modifications in which a part has been removed from the N- and/or C-terminus of a mature protein.

As used herein, the term "conservative substitution" refers to the substitution of an amino acid with another amino acid having similar structural and/or chemical properties. The polypeptide of the present disclosure may have, for example, one or more conservative substitutions, while still retaining one or more biological activities possessed by the polypeptide of SEQ ID NO: 3 or SEQ ID NO: 5. Such amino acid substitution may generally occur based on similarity of polarity, charge, solubility, hydrophobicity, hydrophilicity, and/or amphipathic nature of a residue.

In one embodiment, the polypeptide provided in the present disclosure may comprise a polypeptide consisting of the amino acid sequence of SEQ ID NO: 3 or SEQ ID NO: 5 or an amino acid sequence having at least 60% or more, for example, at least 65%, 70%, 75%, 80%, 85%, 90%, 91%, 92%, 93%, 94%, 95%, 96%, 97%, 98%, or 99% or more homology or identity to the amino acid sequence of SEQ ID NO: 3 or SEQ ID NO: 5, or comprising the amino acid sequence, or essentially consisting of the amino acid sequence. In addition, as long as a polypeptide has the above homology or identity and has fumonisin degradation activity, it is comprised in the polypeptide having fumonisin degradation activity provided in the present disclosure.

As used herein, the term 'homology' or 'identity' refers to a degree of relevance between two given amino acid sequences or nucleotide sequences, and may be expressed as a percentage. The terms 'homology and identity' may often be used interchangeably with each other.

The sequence homology or identity of conserved polynucleotides or polypeptides may be determined by standard alignment algorithms and may be used with a default gap penalty established by the program being used. Substantially, homologous or identical sequences may generally hybridize under moderately or highly stringent conditions to the entire length of the sequence or at least about 50%, 60%, 70%, 80%, or 90% of the full-length. It is apparent that hybridization also comprises hybridization of a polynucleotide with a polynucleotide comprising a general codon or a codon in consideration of codon degeneracy.

Whether or not any two polynucleotide or polypeptide sequences have homology, similarity, or identity may be, for example, determined using a known computer algorithm such as the "FASTA" program using default parameters, as in Pearson et al (1988) [Proc. Natl. Acad. Sci. USA 85]: 2444. Alternatively, it may be determined by the Needleman Wunsch algorithm (Needleman and Wunsch, 1970, J. Mol. Biol. 48: 443-453), as performed using the Needleman program of the EMBOSS package (EMBOSS: The European Molecular Biology Open Software Suite, Rice et al., 2000, Trends Genet. 16: 276-277) (version 5.0.0 or later) (GCG program package (Devereux, J., et al, Nucleic Acids Research 12: 387 (1984)), BLASTP, BLASTN, FASTA (Atschul, [S.] [F.,] [ET AL, J MOLEC BIOL 215]: 403 (1990); Guide to Huge Computers, Martin J. Bishop, [ED.,]Academic Press, San Diego,1994, and [CARILLO et al.](1988) SIAM J Applied Math 48: 1073). For example, the homology, similarity, or identity may be determined using BLAST or ClustalW of the National Center for Biotechnology Information.

The homology, similarity, or identity of polynucleotides or polypeptides may be, for example, determined by comparing sequence information using, for example, the GAP computer program, such as Needleman et al. (1970), J Mol Biol. 48:443, as disclosed in Smith and Waterman, Adv. Appl. Math (1981) 2:482. Briefly, the GAP program defines the homology, similarity, or identity as the value obtained by dividing the number of similarly aligned symbols (i.e., nucleotides or amino acids) by the total number of the symbols in the shorter of the two sequences. Default parameters for the GAP program may comprise (1) a unitary matrix (comprising a value of 1 for identities and 0 for non-identities) and the weighted comparison matrix of Gribskov et al(1986) Nucl. Acids Res. 14: 6745, as disclosed in Schwartz and Dayhoff, eds., Atlas Of Protein Sequence And Structure, National Biomedical Research Foundation, pp. 353-358 (1979) (or EDNAFULL substitution matrix (EMBOSS version of NCBI NUC4.4)); (2) a penalty of 3.0 for each gap and an additional 0.10 penalty for each symbol in each gap (or a gap opening penalty of 10 and a gap extension penalty of 0.5); and (3) no penalty for end gaps.

Further, whether or not any two polynucleotide or polypeptide sequences have homology, similarity, or identity with each other may be identified by comparing the sequences in a Southern hybridization experiment under stringent conditions as defined, and appropriate hybridization conditions defined are within the skill of the art, and may be determined by a method well known to those skilled in the art (e.g., J. Sambrook et al., Molecular Cloning, A Laboratory Manual, 2nd Edition, Cold Spring Harbor Laboratory Press, Cold Spring Harbor, New York, 1989; F.M. Ausubel et al., Current Protocols in Molecular Biology, John Wiley & Sons, Inc., New York).

Another aspect of the present disclosure provides a polynucleotide encoding the polypeptide having fumonisin degradation activity of the present disclosure.

As used herein, the term "polynucleotide", which is a long chain polymer of nucleotides formed by linking nucleotide monomers via covalent bonds, refers to a DNA or RNA strand having a predetermined length or more.

The polynucleotide encoding the polypeptide having fumonisin degradation activity of the present disclosure may comprise any polynucleotide without limitation, as long as it is a polynucleotide encoding the polypeptide of SEQ ID NO: 3 or SEQ ID NO: 5 or a polypeptide having activity corresponding thereto. For example, the polynucleotide encoding the polypeptide having fumonisin degradation activity of the present disclosure may be a polynucleotide sequence encoding the amino acid sequence of SEQ ID NO: 3 or SEQ ID NO: 5 or a polypeptide having at least 60% or more homology or identity thereto.

The polynucleotide encoding the polypeptide having fumonisin degradation activity of the present disclosure may undergo various modifications in the coding region within the scope that does not change the amino acid sequence of the polypeptide, due to codon degeneracy or in consideration of the codons preferred in an organism in which the polypeptide is to be expressed.

In one embodiment, the polynucleotide encoding the polypeptide having fumonisin degradation activity of the present disclosure may consist of or essentially consist of a nucleotide sequence having at least 70%, 75%, 80%, 85%, 90%, 91%, 92%, 93%, 94%, 95%, 96%, 97%, 98%, or 99% or more homology or identity to a sequence of SEQ ID NO: 4 or SEQ ID NO: 6, but is not limited thereto. Further, the polynucleotide of the present disclosure may comprise a probe that may be prepared from a known gene sequence, e.g., any sequence capable of hybridizing with a sequence complementary to all or part of the polynucleotide sequence of the present disclosure under stringent conditions without limitation.

The term "stringent conditions" refers to conditions under which specific hybridization between polynucleotides is allowed. Such conditions are specifically described in the literature (see J. Sambrook et al., Molecular Cloning, A Laboratory Manual, 2nd Edition, Cold Spring Harbor Laboratory Press, Cold Spring Harbor, New York, 1989; F.M. Ausubel et al., Current Protocols in Molecular Biology, John Wiley & Sons, Inc., New York, 9.50-9.51, 11.7-11.8). For example, the stringent conditions may comprise conditions under which polynucleotides having high homology or identity, polynucleotides having 70% or more, 75% or more, 80% or more, 85% or more, 90% or more, 95% or more, 96% or more, 97% or more, 98% or more, or 99% or more homology or identity are hybridized with each other, and polynucleotides having homology or identity lower than the above are not hybridized with each other, or washing conditions of common Southern hybridization, i.e., washing once, specifically, twice or three times at a salt concentration and a temperature corresponding to 60°C, 1xSSC, 0.1% SDS, specifically, 60°C, 0.1xSSC, 0.1% SDS, and more specifically 68°C, 0.1xSSC, 0.1% SDS.

Hybridization requires that two nucleic acids have complementary sequences, although mismatches between bases are possible depending on the stringency of the hybridization. The term "complementary" is used to describe the relationship between nucleotide bases that may hybridize with each other. For example, with respect to DNA, adenine is complementary to thymine, and cytosine is complementary to guanine. Therefore, the polynucleotide of the present disclosure may comprise isolated nucleic acid fragments complementary to the entire sequence as well as nucleic acid sequences substantially similar thereto.

Specifically, polynucleotides having homology or identity to the polynucleotide of the present disclosure may be detected using hybridization conditions comprising a hybridization step at a Tm value of 55°C under the above-described conditions. Further, the Tm value may be 60°C, 63°C, or 65°C, but is not limited thereto, and may be appropriately adjusted by those skilled in the art depending on the purpose thereof.

The appropriate stringency for hybridizing the polynucleotides depends on the length of the polynucleotides and the degree of complementation, and these variables are well known in the art (e.g., J. Sambrook et al., supra).

Still another aspect of the present disclosure provides a vector comprising the polynucleotide encoding the polypeptide having fumonisin degradation activity of the present disclosure. The polypeptide and polynucleotide are as described in other aspects.

As used herein, the term "vector" refers to a DNA construct comprising the nucleotide sequence of the polynucleotide encoding the desired polypeptide operably linked to a suitable expression regulatory region (expression regulatory sequence) so as to express the desired polypeptide in a suitable host cell. The expression regulatory region may comprise a promoter capable of initiating transcription, any operator sequence for regulating the transcription, a sequence encoding a suitable mRNA ribosome-binding site, and a sequence for regulating termination of transcription and translation. Once transformed into a suitable host cell, the vector may replicate or function independently from the host genome, or may integrate into the genome thereof.

For example, the polynucleotide encoding the desired protein may be expressed in the chromosome through a vector for intracellular chromosomal insertion. The insertion of the polynucleotide into the chromosome may be performed by any method known in the art, e.g., by homologous recombination, but is not limited thereto. The vector may further comprise a selection marker to confirm the insertion into the chromosome. The selection marker is for selecting the cells transformed with the vector, i.e., for confirming whether the desired nucleic acid molecule has been inserted, and markers that provide selectable phenotypes, such as drug resistance, auxotrophy, resistance to cytotoxic agents, or expression of surface proteins, may be used. Only cells expressing the selection marker are able to survive or exhibit different phenotypes under the environment treated with the selective agent, and thus the transformed cells may be selected.

The vector used in the present disclosure is not particularly limited, and any vector known in the art may be used.

Examples of the vectors commonly used in prokaryotic cells may comprise, as phage vectors or cosmid vectors, pWE15, M13, MBL3, MBL4, IXII, ASHII, APII, t10, t11, Charon4A, and Charon21A, etc., and as plasmid vectors, those based on pBR, pUC, pBluescriptII, pGEM, pTZ, pCL and pET, etc. Specifically, pDZ, pACYC177, pACYC184, pCL, pECCG117, pUC19, pBR322, pMW118, pCC1BAC, pDCM2 vector, etc. may be used.

Examples of the vectors used in eukaryotic cells may comprise, as yeast expression vectors, integrative yeast plasmids (YIp) and extrachromosomal plasmid vectors. The extrachromosomal plasmid vectors may comprise episomal yeast plasmids (YEp), replicative yeast plasmids (YRp), and yeast centromeric plasmids (YCp). Further, artificial yeast chromosomes (YACs) may also be used as the vector of the present disclosure. Specific examples of the available vectors may comprise pESCHIS, pESC-LEU, pESC-TRP, pESC-URA, Gateway pYES-DEST52, pAO815, pGAPZ A, pGAPZ B, pGAPZ C, pGAPα A, pGAPα B, pGAPα C, pPIC3.5K, pPIC6 A, pPIC6 B, pPIC6 C, pPIC6α A, pPIC6α B, pPIC6α C, pPIC9K, pYC2/CT, pYD1 Yeast Display Vector, pYES2, pYES2/CT, pYES2/NT A, pYES2/NT B, pYES2/NT C, pYES2/CT, pYES2.1, pYES-DEST52, pTEF1/Zeo, pFLD1, PichiaPinkTM, p427-TEF, p417-CYC, pGAL-MF, p427-TEF, p417-CYC, PTEF-MF, pBY011, pSGP47, pSGP46, pSGP36, pSGP40, ZM552, pAG303GAL-ccdB, pAG414GAL-ccdB, pAS404, pBridge, pGAD-GH, pGAD T7, pGBK T7, pHIS-2, pOBD2, pRS408, pRS410, pRS418, pRS420, pRS428, yeast micron A form, pRS403, pRS404, pRS405, pRS406, pYJ403, pYJ404, pYJ405 and pYJ406, but are not limited thereto.

As used herein, the term "transformation" means that a vector comprising a polynucleotide encoding a target protein is introduced into a host cell or a microorganism so that the protein encoded by the polynucleotide may be expressed in the host cell. The transformed polynucleotide may be located regardless of the position, either by being inserted into the chromosome of the host cell or located outside the chromosome as long as it may be expressed in the host cell. Further, the polynucleotide comprises DNA and RNA encoding a desired protein. The polynucleotide may be introduced in any form as long as it may be introduced into a host cell and expressed. For example, the polynucleotide may be introduced into a host cell in the form of an expression cassette, which is a gene construct comprising all elements required for self-expression. The expression cassette may usually comprise a promoter operably linked to the polynucleotide, a transcription termination signal, a ribosome binding site, and a translation termination signal. The expression cassette may be in the form of an expression vector capable of self-replicating. Further, the polynucleotide may be introduced into a host cell in its own form and operably linked to a sequence required for expression in the host cell, but is not limited thereto.

Further, the term "operably linked" means that the gene sequence is functionally linked to a promoter sequence that initiates and mediates transcription of the polynucleotide encoding the desired polypeptide of the present disclosure.

The method of transforming the vector of the present disclosure comprises any method of introducing the nucleic acid into a cell, and may be performed by selecting a suitable standard technique known in the art according to the host cell. For example, the transformation method may comprise electroporation, calcium phosphate (CaPO₄) precipitation, calcium chloride (CaCl₂) precipitation, microinjection, a polyethylene glycol (PEG) technique, a DEAE-dextran technique, a cationic liposome technique, a lithium acetate-DMSO technique, etc., but is not limited thereto.

Still another aspect of the present disclosure provides a host cell expressing the polypeptide having fumonisin degradation activity of the present disclosure.

**The** host cell may be a host cell comprising one or more of the polypeptide having fumonisin degradation activity of the present disclosure; the polynucleotide encoding the polypeptide; and the vector comprising the polynucleotide.

**In** one embodiment, the vector may be integrated into the chromosome as described above, or may remain as a self-replicating extrachromosomal vector.

**The** host cell of the present disclosure may be any cell useful in the recombinant production of the polypeptide having fumonisin degradation activity, for example, a prokaryotic or eukaryotic cell. For example, the host cell may be a fungal cell. For example, the prokaryotic host cell may be any gram-positive or gram-negative bacterium.

For example, the host cell may be a microorganism. For example, the host cell may be *Escherichia coli* (*E. coli*), but is not limited thereto.

Still another aspect of the present disclosure provides a composition for degrading fumonisin, the composition comprising any one or more of the polypeptide having fumonisin degradation activity of the present disclosure; the polynucleotide encoding the polypeptide; the vector comprising the polynucleotide; and the host cell expressing the polypeptide having fumonisin degradation activity.

The polypeptide having fumonisin degradation activity of the present disclosure; the polynucleotide encoding the polypeptide; the vector comprising the polynucleotide; and/or the host cell expressing the polypeptide having fumonisin degradation activity may be used in degrading fumonisin.

In one embodiment, the fumonisin may be present in food.

In one embodiment, the fumonisin may be present in feed.

The composition of the present disclosure may be used to degrade and detoxify fumonisin present in food and/or feed.

Accordingly, still another aspect of the present disclosure provides a composition for detoxifying fumonisin present in food, feed, or both thereof, the composition comprising any one or more of the polypeptide having fumonisin degradation activity of the present disclosure; the polynucleotide encoding the polypeptide; the vector comprising the polynucleotide; and the host cell expressing the polypeptide having fumonisin degradation activity.

Still another aspect of the present disclosure provides a feed additive composition comprising any one or more of the polypeptide having fumonisin degradation activity of the present disclosure; the polynucleotide encoding the polypeptide; the vector comprising the polynucleotide; and the host cell expressing the polypeptide having fumonisin degradation activity.

In one embodiment, the composition of the present disclosure may further comprise a naturally occurring substance or a non-naturally occurring substance.

Examples of the substances that may be added comprise stabilizers, surfactants, builders, chelating agents, dispersants, enzymes, enzyme stabilizers, catalysts, activators, carriers, binders, lubricants, disintegrants, excipients, solubilizers, suspending agents, colorants, flavoring agents, buffers, preservatives, analgesics, solubilizers, isotonic agents, stabilizers, diluents, lubricants, preservatives, etc., but are not limited thereto.

Still another aspect of the present disclosure provides a method of degrading fumonisin, the method comprising the step of bringing fumonisin into contact with the polypeptide having fumonisin degradation activity of the present disclosure; and/or the host cell expressing the polypeptide having fumonisin degradation activity.

Still another aspect of the present disclosure provides a method of preparing the polypeptide having fumonisin degradation activity, the method comprising the step of culturing a host cell comprising any one or more of the polypeptide having fumonisin degradation activity of the present disclosure, the polynucleotide encoding the polypeptide, and the vector comprising the polynucleotide.

As used herein, the term "culturing" means that the host cell is grown under appropriately controlled environmental conditions. **The** culturing process of the present disclosure may be performed in a suitable medium and culture conditions known in the art. Such a culturing process may be easily adjusted for use by those skilled in the art according to the strain to be selected. Specifically, the culturing may be a batch culture, a continuous culture, or a fed-batch culture, but is not limited thereto.

As used herein, the term "medium" refers to a mixture of materials which comprises nutrient materials required for culturing the host cell as a main ingredient, and it supplies nutrient materials and growth factors, along with water that is essential for survival and growth. Specifically, the medium and other culture conditions used for culturing the host cell of the present disclosure may comprise any medium commonly used for culturing host cells without any particular limitation. However, the host cell of the present disclosure may be cultured under aerobic conditions in a common medium comprising appropriate carbon sources, nitrogen sources, phosphorus sources, inorganic compounds, amino acids, and/or vitamins, while adjusting temperature, pH, etc.

In one embodiment, the preparation method may further comprise the step of recovering the polypeptide having fumonisin degradation activity that is expressed in the culturing step.

In the recovering step, the polypeptide may be recovered using a method known in the art to which the present disclosure belongs. For example, the polypeptide may be recovered from a nutrient medium by common procedures comprising, but not limited to, collection, centrifugation, filtration, extraction, spray-drying, evaporation or precipitation.

The recovering method may be collecting the polypeptide using the method of culturing the host cell of the present disclosure, for example, using a suitable method known in the art according to a batch culture, continuous culture, or fed-batch culture method. For example, methods such as centrifugation, filtration, treatment with a protein crystallizing precipitant (salting-out method), extraction, sonication, ultrafiltration, dialysis, various kinds of chromatographies such as molecular sieve chromatography (gel filtration), adsorption chromatography, ion exchange chromatography, affinity chromatography, etc., HPLC and a combination thereof may be used, and the polypeptide may be recovered from the medium or the host cell using suitable methods known in the art.

In another embodiment, the polypeptide expressed by the host cell in the culturing step may not be recovered. The host cell itself expressing the polypeptide may be used as a source of the polypeptide.

Still another aspect of the present disclosure provides use of the polypeptide of SEQ ID NO: 3 or SEQ ID NO: 5, or a polypeptide having 75% or more sequence identity thereto as a fumonisin degradation enzyme.

### [Mode for Carrying Out the Invention]

Hereinafter, the present disclosure will be described in more detail with reference to Examples and Experimental Examples. However, these Examples and Experimental Examples are only for illustrating the present disclosure, and the scope of the present disclosure is not intended to be limited by these Examples and Experimental Examples.

### Example 1. Construction of Vector Expressing Novel Fumonisin Degradation Enzyme

Polynucleotides (SEQ ID NOS: 2, 4, and 6) each encoding a carboxylesterase family protein derived from *Bradyrhizobium* sp. (hereinafter, referred to as EFB1-9, SEQ ID NO: 1) and two types of variants comprising multiple mutations (hereinafter, referred to as CB1 and CB4, SEQ ID NOS: 3 and 5) were synthesized by Cosmo Genetech, and cloned into a pET vector (Novagen) using primers listed in Table 1.

**[Table 1]**

| | SEQ ID NO. | Name | Primer sequence (5'→3') |
|---|---|---|---|
| EFB1-9 | 7 | EFB1-9-F | cggcagccatatgGAGCCCGTCATCGTTCAAACTTC |
| | 8 | EFB1-9-R | tcgagtgcggccgcTCACGGTTTGGTTAAGAAACTCTC G |
| CB1 | 9 | CB1-F | ggcagccatatgATGGAACCTGTGATCGTCCAAACC |
| | 10 | CB1-R | tcgagtgcggccgcTCAAGGCTTAGTCAGAAATGACTC GACAAA |
| CB4 | 11 | CB4-F | ggcagccatatgATGGAACCGGTCATCGTTCAGAC |
| | 12 | CB4-R | tcgagtgcggccgcTCACGGCTTCGTAAGGAACGACT C |

In detail, EFB1-9, CB1, and CB4 were prepared by PCR using the gene construct, primers (SEQ ID NOS: 7 and 8, SEQ ID NOS: 9 and 10, SEQ ID NOS: 11 and 12 in Table 1), and a PCR premix (iNtRON, cat no. 25185), respectively. PCR was performed using Eppendorf Mastercycler Nexus GX2, and reaction conditions were as follows.
Initial denaturation - 94°C, 2 min
Denaturation - 94°C, 20 sec
Annealing - 56°C, 10 sec
Extension - 72°C, 2 min (35 cycles from denaturation to extension)
Final extension - 72°C, 5 min

After treating each PCR product and vector with restriction enzymes (Ndel, Notl), ligation was performed using T4 DNA ligase (NEB, Cata# M0202S), and then transformed into *E. coli* Dh5α strain to identify sequence mutations through sequencing.

### Example 2. Comparison of Homology of Multiple Mutations

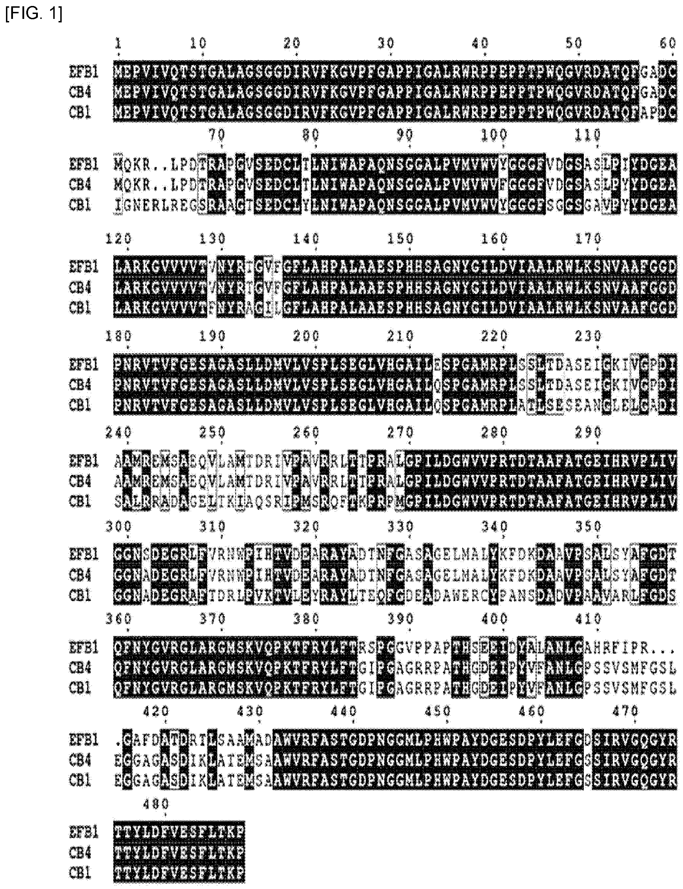

The sequence alignment between EFB1-9, CB1, and CB4 (FIG. 1) was carried out using Clustal Omega and ESPript 3.0, and the sequence homology was obtained using NCBI BLAST, which is shown in Table 2.

**[Table 2]**

| | EFB₁-9 | CB1 | CB4 |
|---|---|---|---|
| EFB₁-9 | 100.0 | | |
| CB1 | 73.4 | 100.0 | |
| CB4 | 92.0 | 81.1 | 100.0 |

### Example 3. Expression and Purification for Assessment of Novel FUM Degradation Enzyme Activity

EFB1-9, CB1, and CB4 prepared in Example 1 were transformed into *E. coli* BL21 (DE3), and inoculated into a sterile LB medium (BD Difco), followed by preculture at 37°C, 200 rpm for 16 hours. Thereafter, 1/100 of the medium volume was inoculated into a flask comprising sterile LB medium, and cultured at 37°C, 200 rpm until the absorbance (OD₆₀₀) became between 0.4 and 0.5. Isopropyl β-D-1-thiogalactopyranoside (IPTG) was added to a final concentration of 1 mM, and further cultured for an additional 16 hours. Then, the cells were recovered by centrifugation. 20 ml of a lysis buffer (50 mM Tris-HCl pH 8.0, 100 mM NaCl, 10 mM imidazole) was added to the recovered cells, followed by resuspending, and then a crude enzyme solution was obtained through sonication and centrifugation. The crude enzyme solution was applied to and adsorbed onto a Ni-NTA resin (Qiagen, Cat no. 30230), and then the enzyme was purified by sequentially applying a washing buffer (20 mM imidazole concentration in the lysis buffer composition) and an elution buffer (250 mM imidazole concentration in the lysis buffer composition).

The protein concentration was determined by mixing 5 µl of the diluted enzyme solution + 250 µl of Bradford solution (Quick Start^{™} Bradford 1x Dye Reagent, #5000205) and measuring absorbance at 595 nm.

### Example 3. Assessment of Activity Using TLC

To analyze the activities of the purified EFB1-9, CB1, and CB4 for FUM, the purified enzyme reaction solution (50 mM Tris-HCl, pH 7.4) was treated with fumonisin (CAS 116355-83-0, Fumonisin B1 from *Fusarium moniliforme*) dissolved in a solution of acetonitrile and distilled water at a ratio of 1:1, and allowed to react at room temperature for 1 hour and then left at 95°C for 10 minutes to stop the reaction.

The reaction product was spotted onto one end of a TLC plate (Merck, TLC Silica gel 60 F254 20X20cm, Cat# 105554) coated with silica gel on an aluminum surface and placed vertically in a sealed chamber comprising a developing solution of acetonitrile and distilled water at a ratio of 7:3 for 6 minutes and 30 seconds. The plate was dried and then immersed in 0.2% ninhydrin developer dissolved in ethanol and dried in a dryer at 140°C for 10 minutes.

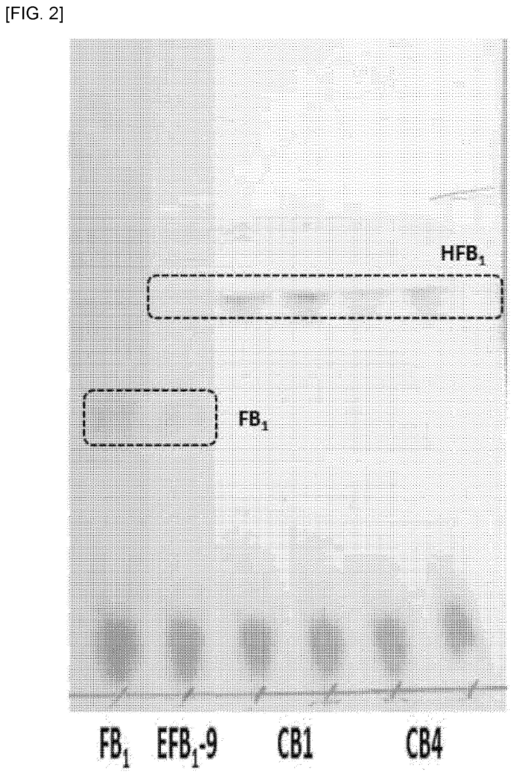

As a result, it was found that the FB1 spot (Rf=0.59) became lighter and an HFB1 spot (Rf=0.81) appeared on the plate, as compared to a control group. The detailed results are as shown in FIG. 2 and Table 3.

**[Table 3]**

| No. | Sample | FB1 | HFB1 |
|---|---|---|---|
| 1 | Negative control | O | X |
| 2 | EFB1-9 | O | Δ |
| 3 | CB1 | X | O |
| 4 | CB4 | X | O |

### Example 4. Changes in Fumonisin B1 Content according to Reaction Time

For comparative assessment of the activities of the purified EFB1-9, CB1, and CB4 for fumonisin B1, the purified enzyme reaction solution (50 mM Tris-HCl, pH 7.4) with a protein concentration of 0.1 mg/ml was treated with fumonisin (CAS 116355-83-0, Fumonisin B1 from *Fusarium moniliforme*) dissolved in a solution of acetonitrile and distilled water at a ratio of 1:1, and allowed to react at room temperature for 10 minutes, 1 hour, and 24 hours, and then left at 95°C for 10 minutes to stop the reaction.

Each sample was analyzed for the content of fumonisin B1 through liquid chromatography-fluorescence detection (LC-FLD). Liquid chromatography-fluorescence detection was performed using Acquity UPLC and a fluorescence detector from Waters. Fumonisin B1 was separated by a column and then analyzed by post-column derivatization with an o-phthalaldehyde (OPA) reagent, before entering the fluorescence detector. The detailed analysis conditions were as follows.
(1) Chromatography: Waters Acquity UPLC System
(2) Column: Waters Acquity UPLC BEH C18 1.7 µm 2.1x150 mm
(3) Column temperature: 40°C
(4) Flow rate: 0.25 mL/min
(5) Sample injection: 10.0 µL
(6) Mobile phase:
A: 13.7 mM Octanesulfonic acid + 25 mM Potassium dihydrogen phosphate in DW (pH 2.1, by H₃PO₄),
B: 13.7 mM Octanesulfonic acid + 25 mM Potassium dihydrogen phosphate in 50% Acetonitrile (pH 2.1, by H₃PO₄)

(7) Elution conditions:

**[Table 4]**

| Time (min) | %A | %B |
|---|---|---|
| 0.0 | 25 | 75 |
| 8.0 | 25 | 75 |
| 8.1 | 0 | 100 |
| 10.5 | 0 | 100 |
| 10.6 | 25 | 75 |
| 16.0 | 25 | 75 |

(8) Detection wavelength
Excitation wavelength 338 nm, Emission wavelength 425 nm
(9) Post-column derivatization method
Reagent: 5.2 mM o-phthalaldehyde (OPA) in borate buffer
Reagent flow rate: 0.25 mL/min
Reactor temperature: 40°C

Through the reaction of purified EFB1-9, CB1, and CB4, it was confirmed that the concentration of fumonisin B1 decreased with the reaction time. In particular, it was confirmed that when CB1 and CB4 were used, fumonisin B1 used as a substrate completely disappeared after 10 minutes and 30 minutes of reaction. These results confirmed that EFB1-9, CB1, and CB4 all have fumonisin B1 degradation activity, and CB1 and CB4 also have improved activity for fumonisin B1 through multiple mutations, as compared to EFB1-9.

### Example 5. Analysis of Reaction Patterns according to Reaction Time

For comparative assessment of the reaction patterns of the purified EFB1-9, CB1, and CB4 for fumonisin B1, the purified enzyme reaction solution (50 mM Tris-HCl, pH 7.4) was treated with fumonisin (CAS 116355-83-0, Fumonisin B1 from *Fusarium moniliforme*) dissolved in a solution of acetonitrile and distilled water at a ratio of 1:1, and allowed to react for 30 minutes, and then left at 95°C for 10 minutes to stop the reaction. Each sample was analyzed for the content of fumonisin B1 through LC-FLD, and the ratio of the reaction product was analyzed by liquid chromatography-time-of-flight mass spectrometry (LC-TOF/MS). The LC-FLD analysis conditions are as in Example 4. LC-TOF/MS analysis was performed using an Acquity UPLC, Xe-vo G2-XS Q-Tof mass spectrometer from Waters, and the analysis conditions were as follows.
(1) Chromatography: Waters Acquity UPLC System
(2) Column: Waters Acquity UPLC BEH C18 1.7 µm 2.1x150 mm
(3) Column temperature: 40°C
(4) Flow rate: 0.20 mL/min
(5) Sample injection: 1.0 µL
(6) Mobile phase:
A: 5 mM Ammonium Formate w. 0.1% Formic acid in DW
B: 5 mM Ammonium Formate w. 0.1% Formic acid in Methanol

(7) Elution conditions:

**[Table 5]**

| Time (min) | %A | %B |
|---|---|---|
| 0.0 | 45 | 55 |
| 6.2 | 45 | 55 |
| 8.2 | 0 | 100 |
| 9.2 | 0 | 100 |
| 9.3 | 45 | 55 |
| 13.0 | 45 | 55 |

(8) Mass spectrometer: Waters Xe-vo G2-XS Q-Tof
Ionization mode: ESI Positive
Capillary voltage: 2.5 kV
Cone voltage: 30 V
Source Temperature: 120 °C
Desolvation Temperature: 400 °C
Mass scan range: 50 - 1000 m/z

The results of analyzing the reaction product of the purified EFB1-9, CB1, or CB4 with fumonisin B1 showed that EFB1-9 immediately produced HFB1, but CB1 and CB4 first accumulated pHFB1, which was then ultimately degraded into HFB1.

**[Table 6]**

| **Sample info** | **LC-FLD** | **LC-TOF/MS (%Area)** | | |
|---|---|---|---|---|
| | **FB1 (ppm)** | **m/z 722.39** (FB₁) | **m/z 564.36** (pHFB₁) | **m/z 406.35** (HFB₁) |
| FB1 | 6.4 | 100.0% | nd | nd |
| EFB1-9 | 4.6 | 70.0% | nd | 30.0% |
| CB1 | 1.95 | 28.0% | 70.0% | 1.3% |
| CB4 | 3.88 | 63.0% | 23.0% | 14.0% |

Based on the above description, it will be understood by those skilled in the art that the present disclosure may be implemented in a different specific form without changing the technical spirit or essential characteristics thereof. **In** this regard, it should be understood that the above embodiment is not limitative, but illustrative in all aspects. The scope of the disclosure is defined by the appended claims rather than by the description preceding them, and therefore all changes and modifications that fall within metes and bounds of the claims, or equivalents of such metes and bounds are therefore intended to be embraced by the claims.

## Claims

1. A polypeptide having fumonisin degradation activity, the polypeptide having SEQ ID NO: 3 or SEQ ID NO: 5 or 75% or more sequence identity thereto.

2. A polynucleotide encoding the polypeptide of claim 1.

3. A vector comprising the polynucleotide of claim 2.

4. A host cell comprising any one or more of a polypeptide having fumonisin degradation activity, the polypeptide having SEQ **ID** NO: 3 or SEQ **ID** NO: 5 or 75% or more sequence identity thereto, a polynucleotide encoding the polypeptide, and a vector comprising the polynucleotide.

5. A composition for degrading fumonisin, the composition comprising any one or more of:
a polypeptide having SEQ **ID** NO: 3 or SEQ **ID** NO: 5 or 75% or more sequence identity thereto;
a polynucleotide encoding the polypeptide;
a vector comprising the polynucleotide; and
a host cell expressing the polypeptide.

6. A composition for detoxifying fumonisin present in food, feed, or both thereof, the composition comprising any one or more of:
a polypeptide having SEQ ID NO: 3 or SEQ ID NO: 5 or 75% or more sequence identity thereto;
a polynucleotide encoding the polypeptide;
a vector comprising the polynucleotide; and
a host cell expressing the polypeptide.

7. A feed additive composition comprising any one or more of:
a polypeptide having SEQ ID NO: 3 or SEQ ID NO: 5 or 75% or more sequence identity thereto;
a polynucleotide encoding the polypeptide;
a vector comprising the polynucleotide; and
a host cell expressing the polypeptide.

8. A method of degrading fumonisin, the method comprising the step of bringing fumonisin into contact with any one or more of a polypeptide having SEQ ID NO: 3 or SEQ ID NO: 5 or 75% or more sequence identity thereto; and a host cell expressing the polypeptide.

9. A method of preparing the polypeptide having fumonisin degradation activity of claim 1, the method comprising the step of:
culturing a host cell comprising any one or more of a polypeptide having SEQ ID NO: 3 or SEQ ID NO: 5 or 75% or more sequence identity thereto, a polynucleotide encoding the polypeptide, and a vector comprising the polynucleotide.

10. Use of a polypeptide having SEQ ID NO: 3 or SEQ ID NO: 5 or 75% or more sequence identity thereto as a fumonisin degradation enzyme.
